Europäisches Patentamt

European Patent Office

Office européen des brevets

(11) Veröffentlichungsnummer: **0 425 672 A1**

(12) **EUROPÄISCHE PATENTANMELDUNG**
veröffentlicht nach Art. 158 Abs. 3
EPÜ

(21) Anmeldenummer: 89909905.5

(22) Anmeldetag: 21.04.89

(86) Internationale Anmeldenummer:
PCT/SU89/00109

(87) Internationale Veröffentlichungsnummer:
WO 90/12568 (01.11.90 90/25)

(51) Int. Cl.5: **A61K 9/52**

(43) Veröffentlichungstag der Anmeldung:
08.05.91 Patentblatt 91/19

(84) Benannte Vertragsstaaten:
**AT DE FR GB IT NL**

(71) Anmelder: **MOSKOVSKY AVTOMOBILESTROITELNY INSTITUT (VTUZ-ZIL)**
ul. Avtozavodskaya, 16
Moscow, 109268(SU)

Anmelder: **VSESOJUZNY NAUCHNO-ISSLEDOVATELSKY INSTITUT GELMINTOLOGII IMENI AKADEMIKA K.I. SKRYABINA**
ul. B.Cheremushkinskaya, 28
Moscow, 117259(SU)

Anmelder: **VSESOJUZNY NAUCHNO-ISSLEDOVATELSKY INSTITUT MEDITSINSKIKH POLIMEROV**
Nauchny proezd, 10
Moscow, 117246(SU)

(72) Erfinder: **GRIGORIANTS, Igor Konstantinovich**
Leninsky pr., 69-135
Moscow, 117296(SU)
Erfinder: **TRIKHANOVA, Galina Andreevna**
ul. Rokotova, 7-2-86
Moscow, 117593(SU)
Erfinder: **BALABUSHEVICH, Alexandr Georgievich**
ul. Isakovskogo, 26-2-68

Moscow, 123631(SU)
Erfinder: **LYAKHNOVICH, Sergei Vladimirovich**
Lomonosovsky pr., 18-132
Moscow, 217296(SU)
Erfinder: **LIPANOV, Alexei Matveevich**
ul. B.Gruzinskaya, 39-122
Moscow, 123056(SU)
Erfinder: **ARBUZOVA, Larisa Alexandrovna**
Orekhovy bulvar, 39-2-417
Moscow, 115573(SU)
Erfinder: **DEMIDOV, Nikolai Vasilievich**
Sevastopolsky pr., 14-188
Moscow, 113447(SU)
Erfinder: **BOCHAROV, Mikhail Yakovlevich**
ul. Sudostroitelnaya, 28-7
Moscow, 115407(SU)
Erfinder: **SHUMAKOVICH, Irina Evgenievna**
Studeny proezd, 2-8-340
Moscow, 129282(SU)
Erfinder: **STUKALOVA, Nadezhda Pavlovna**
ul. Turistskaya, 25-1-52
Moscow, 123514(SU)
Erfinder: **ZAVYALOV, Boris Ivanovich**
ul. Musy Dzhalilya, 16/2-245
Moscow, 115573(SU)
Erfinder: **MITUKHIN, Igor Vasilievich**
ul. Bogdanova, 36-48
Moscow, 119618(SU)

(74) Vertreter: **Nix, Frank Arnold, Dr.**
Kröckelbergstrasse 15
W-6200 Wiesbaden(DE)

EP 0 425 672 A1

(54) **EINRICHTUNG ZUR KONTROLLIERBAREN DOSIERTEN EINFUEHRUNG VON WIRKSTOFFEN IN EIN FUNKTIONIERUNGSMEDIUM.**

(57) Die Einrichtung zur kontrollierbaren dosierten Einführung von Wirkstoffen in ein Funktionierungsmedium enthält ein hohles zylindrisches Gehäuse (I), welches trennbar mindestens mit einer Trennungsebene ausgeführt ist, die längs einer Mantellinie des zylinrischen Gehäuses (I) verlauft. In dieser Trennungsebene liegt eine Trennwand (2), die das zylindrische Gehäuse (I) in Teile (3) trennt, die in bezug auf das Funktionierungsmedium hermetisch abgeschlossen sind. Jeder Teil (3) besitzt mindestens ein Mittel zum Verbinden des zylindrischen Gehäuses (I) mit dem Funktionierungsmedium. Die Teile (3) des Gehäuses (I) sind durch eine Torsionsfeder (4) miteinander verbunden, deren Achse zur Längsachse des Gehäuses (I) senkrecht ist. Die Torsionsfeder (4) ist mit der Möglichkeit einer Drehung der Teile (3) des Gehäuses (I) relativ zueinander in einer zur Längsachse des Gehäuses (I) parallelen Ebene im Augenblick des Einbringens des Gehäuses (I) in das Funktionierungsmedium angebracht. Die Einrichtung ist ferner mit zumindest einem Fixator (5) aus einem löslichen Material ausgestattet, der an der Aussenfläche des Gehäuses (I) angebracht ist.

FIG.1

# EINRICHTUNG ZUR KONTROLLIERBAREN DOSIERTEN EINFUHRUNG VON WIRKSTOFFEN IN EIN FUNKTIONIERUNGSMEDIUM

Technisches Gebiet

Die Erfindung bezieht sich auf Einrichtungen zur Einführung von festen, flüssigen und gasförmigen Heilmitteln und anderen Stoffen in den Organismus, insbesondere auf eine Einrichtung zur kontrolierbaren dosierten Einführung von Wirkstoffen in ein Funktionierungsmedium.

Zugrundeliegender Stand der Technik

Bekannt ist ein Absonderungssystem der kontrollierter Zuführung (US, A, 4220I53), das einen zylindrischen Behälter mit einem Heilstoff enthält, dessen Wand aus einem porösen Gewebematerial ausgeführt ist, wobei die Poren desselben mit Hydrogel gefüllt sind. Über die Hydrogelschicht diffundiert die Flüssigkeit aus dem Aussenmedium zum Heilstoff, in das Aussenmedium zurück diffundiert der Heilstoff in Form einer Lösung. Mit Hilfe dieses Systems ist es unmöglich, ein Heilmittel über eine längere Zeit als 120 Tage einzuführen und auch eine dosierte Einführung von zwei miteinander nicht kompatiblen Heilstoffen vorzunehmen.

Bekannt ist ferner eine Pille mit Netzumhüllung (US, A, 4326522), die aus zylindrischen Kapseln besteht, die aus einem biologisch abbaubaren Polymer in einem Gemisch mit einem Heilstoff und einem Beschwerungsmittel ausgeführt sind. Die zylindrischen Kapseln sind durch elastische Federelemente miteinander verbunden, die es der Pille ermöglichen, bei deren Eintritt in den Magen der Wiederkäuer eine solche Form anzunehmen, die das Herausführen der Pille aus dem Magen der Wiederkäuer verhindert. Die gesamte Pille ist von einem Netz umhüllt, das die unter der Einwirkung der Magenflüssigkeit zerfallende Polymermatrix mit dem Wirkstoff zusammenhält. Diese Pille gewährleistet kein kontrollierbares Einführen von Heilstoffen und ermöglicht es nur, einen einzigen Wirkstoff im Verlaufe von 30 Tagen einzuführen.

Bekannt ist eine Einrichtung zur kontrolierbaren dosierten Einführung von Wirkstoffen in ein Funktionierungsmedium (EP, B, 0079724), die ein hohles zylinderförmiges Gehäuse mit einem Mittel zum Verbinden des zylindrischen Gehäuses mit dem Funktionierungsmedium enthält. Am Gehäuse sind flexible Fixatoren angebracht. Vor der Einführung der Einrichtung in den Pansen eines Wiederkäuers werden die Fixatoren entlang der Mantellinie des Gehäuses angeordnet, während sie sich im Innern des Magens dank ihrer Flexibilität unter einem Winkel lagern, der innerhalb einer Spanne von 75-90° in bezug auf die Längsachse des Gehäuses liegt. Dies bewirkt das Festhalten der Einrichtung im Pansen während der gesamten Funktionsdauer derselben. In einer der Stirnseiten des Gehäuses ist ein Mittel zum Verbinden des Gehäuses mit dem Funktionierungsmedium ausgeführt. Im Innern des Gehäuses ist eine Feder angebracht, die auf der einen Seite an der Gehäusestirn befestigt ist, auf der anderen aber an einem Kolben anliegt, der die Fortbewegung des Wirkstoffes zum Mittel zum Verbinden des Gehäuses mit dem Funktionierungsmedium bewirkt. Unter der Einwirkung der Magenflüssigkeit wird die Wirkstofflösung über das Mittel zum Verbinden des Gehäuses mit dem Funktionierungsmedium aus dem Gehäuse abgeleitet. Diese Einrichtung gestattet es nicht, mehrere nicht vermischbare Wirkstoffe gleichzeitig und dosiert einzuführen, weil in ihr die Beschickung mit nur einem Wirkstoff vorgesehen ist. Ferner kann diese Einrichtung auch eine Beschädigung der Tiermagenwände wegen einer Vielzahl von flexiblen Fixatoren bewirken, die an der Aussenseite des Gehäuses angebracht sind.

Offenbarung der Erfindung

Der Erfindung liegt die Aufgabe zugrunde, eine Einrichtung zur kontrollierten dosierten Einführung von Wirkstoffen in ein Funktionierungsmedium zu schaffen, die eine solche Ausführung des zylinderförmigen Gehäuses desselben zulässt, dass dadurch die Möglichkeit einer prolongierten dosierten Einführung von gleichzeitig mehreren Wirkstoffen gewährleistet sowie die Wahrscheinlichkeit einer Beschädigung von Tierpansenwänden verringert ist.

Lösung der technischen Aufgabe

Die gestellte Aufgabe ist dadurch gelöst, dass in der Einrichtung zur kontrollierbaren dosierten Einführung von Wirkstoffen in ein Funktionierungsmedium, die ein hohles zylindrisches Gehäuse mit einem Mittel zum Verbinden des zylindrischen Gehäuses mit dem Funktionierungsmedium enthält, erfindungsgemäss das zylindrisches Gehäuse trennbar mit zumindest einer Trennungsebene ausgeführt ist, die entlang einer Gehäusemantellinie geht. In dieser Trennungsebene ist eine Trennwand angebracht, die das zylindrische Gehäuse in inbezug auf das Funktionierungsmedium hermetisch dichte Teile unterteilt. Jeder der Teile des zylindrischen Gehäuses besitzt mindestens ein Mittel zum Verbinden des Gehäuses mit dem Funktionierungsmedium. Die Teile des zylinderförmigen Gehäuses sind durch eine Torsionsfeder miteinander verbunden, deren Achse zur Längsachse des zylinderförmigen Gehäuses senkrecht und die mit der Möglichkeit einer Drehung der erwähnten Teile des zylindrischen Gehäuses im Augenblick des Einbringens des zylindrischen Gehäuses in das Funktionierungsmedium relativ zueinder in einer zur Längsachse des zylinderförmigen Gehäuses parallelenEbene angebracht ist.

Zweckmässigerweise ist die Einrichtung mit mindestens einem Fixator aus einem löslichen Material ausgestattet, der an der Aussenfläche des zylindrischen Gehäuses angebracht ist und die Teile des zylindrischen Gehäuses im zusammengebauten Zustand bis zum Augenblick des Einbringens des zylindrischen Gehäuses in das Funktionierungsmedium zusammenhält.

Zweckmässigerweise erfolgt die Drehung der erwähnten Teile des zylindrischen Gehäuses um einen Winkel innerhalb einer Spanne von l0-90°.

Zweckdienlicherweise ist ferner jeder Teil des zylindrischen Gehäuses mit mindestens einem Einsatzstück versehen, das im Innern eines Teiles des zylindrischen Gehäuses untergebracht und an den Innenwänden desselben befestigt ist, wobei das Einsatzstück das Innenvolumen des zylindrischen Gehäuses in voneinander isolierte Abteilungen trennt.

Das Einsatzstück kann in einer zur Längsachse des zylindrischen Gehäuses senkrechten Ebene angebracht sein.

Das Einsatzstück kann ferner auch in einer zur Längsachse des zylindrischen Gehäuses parallelen Ebene angebracht sein.

Es empfiehlt sich, dass das zylindrische Gehäuse trennbar mit einer Trennungsebene ausgeführt ist und die Trennwand das zylindrische Gehäuse in zwei Teile trennt, wobei eine Drehung derselben um einen Winkel von 90° ausführbar ist.

Zweckmässigerweise kann das zylinderförmige Gehäuse trennbar mit zwei Trennungsebenen ausgeführt sein, wobei die Trennwand das zylindrische Gehäuse in drei Teile trennt und eine Drehung derselben um einen Winkel von 60° ausführbar ist.

Zweckmässigerweise kann ferner das zylindrische Gehäuse trennbar mit drei Trennungsebenen ausgeführt sein, wobei die Trennwand das zylinderförmige Gehäuse in vier Teile trennt und eine Drehung derselben um einen Winkel von 45° ausführbar ist.

Diese Einrichtung zur kontrollierbaren dosierten Einführung von Wirkstoffen in das Funktionierungsmedium ermöglicht es, eine prolongierte dosierte Einführung gleichzeitig von mehreren Wirkstoffen in das Funktionierungsmedium dank einer entsprechenden konstruktiven Ausführung des zylindrischen Gehäuses der Einrichtung zu gewährleisten. Ebenfalls dank den konstruktiven Besonderheiten der Einrichtung, bei denen zusätzliche Fixatoren nicht mehr notwendig sind, wird die Wahrscheinlichkeit einer Beschädigung der Tierpansenwände verringert. Diese Einrichtung verschiebt sich ununterbrochen im gesamten Magenvolumen, was eine gleichmässige Verteilung von Wirkstoffen im Funktionierungsmedium ermöglicht und den durch die Anwendung derselben erzielten. Heil- bzw. Prophylaxeeffekt zu erhöhen erlaubt. Diese Einrichtung ermöglicht es, bei deren Einsatz zur Ausheilung und Prophylaxe von Tieren, die Gewichtszunahme eines jeden Tieres um einen Betrag von 9 bis 27 kg zu vergrössern.

Kurze Beschreibung der Zeichnungen

Im folgenden wird die Erfindung an Hand konkreter Ausführungsbeispiele und der beiliegenden Zeichnungen erläutert; in den Zeichnungen zeigt:

Fig. l die Gesmtansicht einer Einrichtung zur kontrollierbaren dosierten Einführung von Wirkstoffen in ein Funktionierungsmedium im zusammengebauten Zustand bei trennbarer Ausbildung des zylindrischen Gehäuses mit einer Trennungsebene, im teilweisen Längsschnitt, gemäss der Erfindung;

Fig. 2 die Einrichtung zur kontrollierbaren dosierten Einführung von Wirkstoffen, im Querschnitt nach der Linie II-II aus der Fig. I;

Fig. 3 eine Gesamtansicht der Einrichtung zur kontrollierbaren dosierten Einführung von Wirkstoffen im Arbeitszustand bei trennbarer Ausbildung des zylindrischen Gehäuses mit einer Trennungsebene, gemäss der Erfindung;

Fig. 4 die Einrichtung zur kontrollierbaren dosierten Einführung von Wirkstoffen im Querschnitt nach der Linie IV-IV aus der Fig. 3;

Fig. 5 die Gesamtansicht einer Einrichtung zur kontrollierbaren dosierten Einführung von Wirkstoffen im Arbeitszustand bei trennbarer Ausbildung des Gehäuses mit zwei Trennungsebenen, im teilweisen Längsschnitt, der die Anordnung eines Einsatzstückes im Innern eines Gehäuseteiles zeigt, gemäss der Erfindung;

Fig. 6 die Gesamtansicht einer Einrichtung zur kontrollierbaren dosierten Einführung von Wirkstoffen im zusammengebauten Zustand bei trennbarer Ausbildung des Gehäuses mit drei Trennungsebenen, im teilweisen Längsschnitt, gemäss der Erfindung;

Fig. 7 eine Gesamtansicht der Einrichtung zur kontrollierbaren dosierten Einführung von Wirkstoffen im Arbeitszustand bei trennbarer Ausbildung des Gehäuses mit drei Trennungsebenen, gemäss der Erfindung;

Fig. 8 die Gesamtansicht einer Einrichtung zur kontrollierbaren dosierten Einführung von Wirkstoffen im Arbeitszustand mit im Innern der Gehäuseteile untergebrachten Einsatzstücken, im Längsschnitt, gemäss der Erfindung.

Beste Ausführungsform der Erfindung

Die Einrichtung zur kontrollierbaren dosierten Einführung von Wirkstoffen in das Funktionierungsmedium enthält ein hohles zylindrisches Gehäuse I (Fig. I), das trennbar mit einer Trennungsebene ausgeführt ist, die entlang der Mantellinie des zylindrischen Gehäuses 1 verläuft. In dieser Trennungsebene liegt eine Trennwand 2, die das zylindrische Gehäuse I in zwei Teile 3 trennt, die aus einem polymeren bzw. einem anderen biologisch unschädlichen Material ausgeführt sind. Die Teile 3 des Gehäuses I sind durch eine Torsionsfeder 4 miteinander verbunden, deren Achse zur Längsachse das zylindrischen Gehäuses I senkrecht ist. Die Torsionsfeder 4 ist mit der Möglichkeit einer Drehung der Gehäuseteile 3 im Augenblick des Einbringens des Gehäuses I in das Funktionierungsmedium (in Fig. ist das Funktionierungsmedium nicht abgebildet) relativ zueinander in der zur Längsachse des Gehäuses I parallelen Ebene angebracht. Vor dem Einbringen der Einrichtung in den Tiermagen werden die Teile 3 des Gehäuses I auf eine solche Weise aneinandergepasst, dass sie ein einheitliches Ganzes bilden. Zum Festhalten des zylindrischen Gehäuses I im zusammengebauten Zustand sind in der Einrichtung zwei Fixatoren 5 aus einem Material vorgesehen, das unter Einwirkung des Funktionierungsmediums zerstört wird, welche Fixatoren an der Aussenseite des Gehäuses I angeordnet sind. Die Einrichtung kann mindestens einen Fixator 5 aus einem löslichen Material besitzen. In den Wänden eines jeden Teils 3 des Gehäuses I sind zehn Bohrungen 6 ausgeführt, die die Mittel zum Verbinden des Gehäuses I mit dem Funktionierungsmedium darstellen. Der Durchmesser der Bohrungen 6 sowie deren Anzahl sind in jedem konkreten Ausführungsbeispiel konkret gewählt. Jeder Teil 3 des Gehäuses I kann mindestens ein Mittel zum Verbinden des Gehäuses I mit dem Funktionierungsmedium besitzen.

Im Innern der Teile 3 des Gehäuses I ist ein Wirkstoff 7 in Form einer Mischung mit einer polymeren Matrize 8 bzw. in reinem Zustand untergebracht. In jedem Teil 3 des Gehäuses I sind entweder ein und derselbe Wirkstoff 7 oder aber verschiedene Wirkstoffe untergebracht, so beispielsweise in einem Teil 3 des Gehäuses I ist das Antihelminthenpräparat Tividin, in dem anderen Teil 3 aber das Antibiotikum Vitatetrin untergebracht.

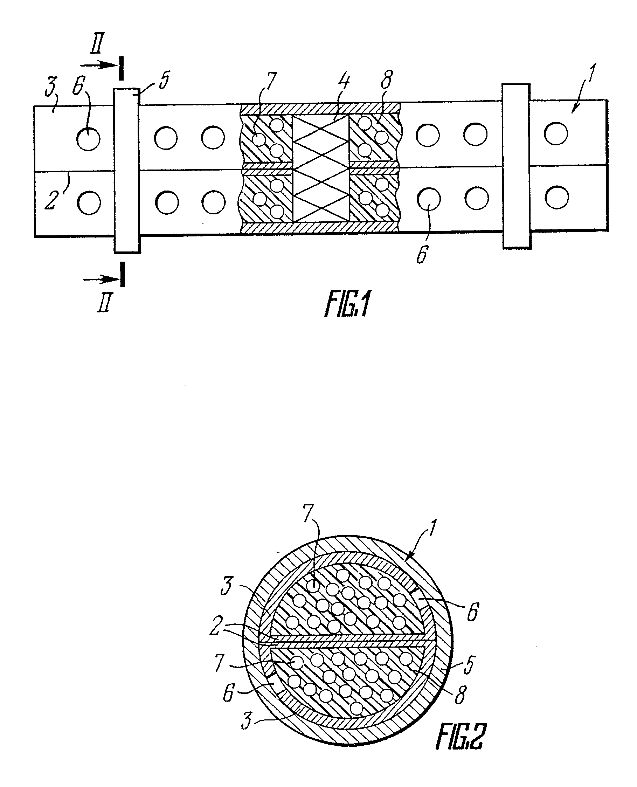

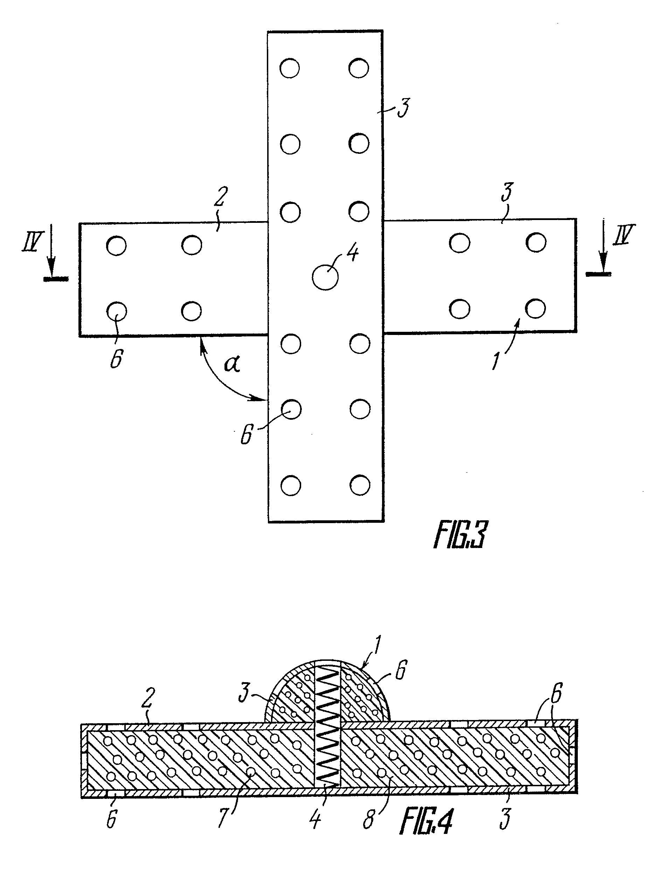

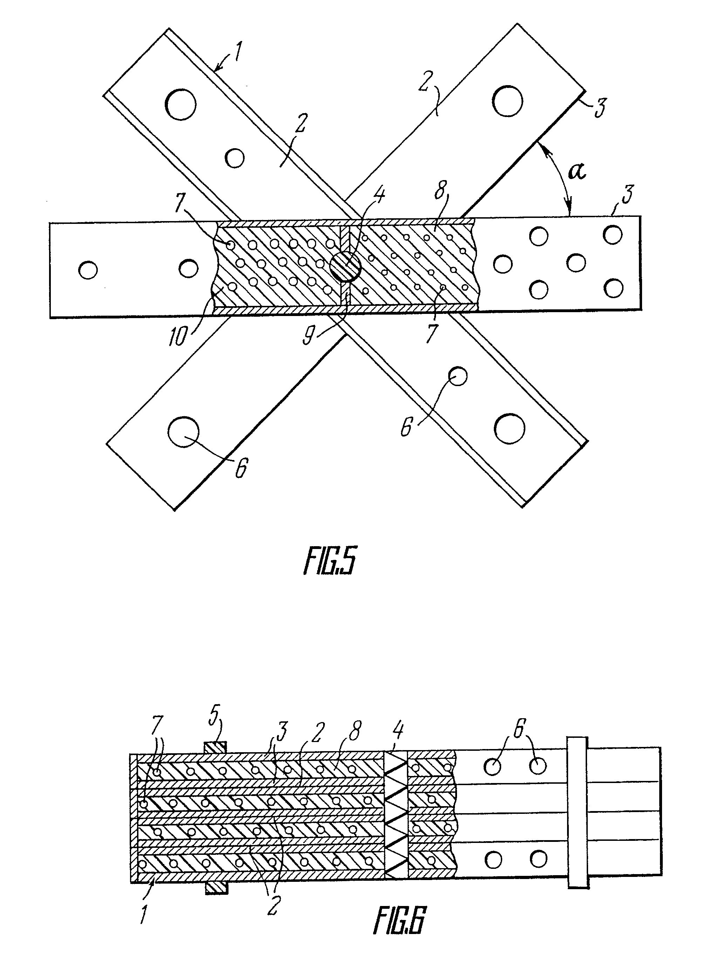

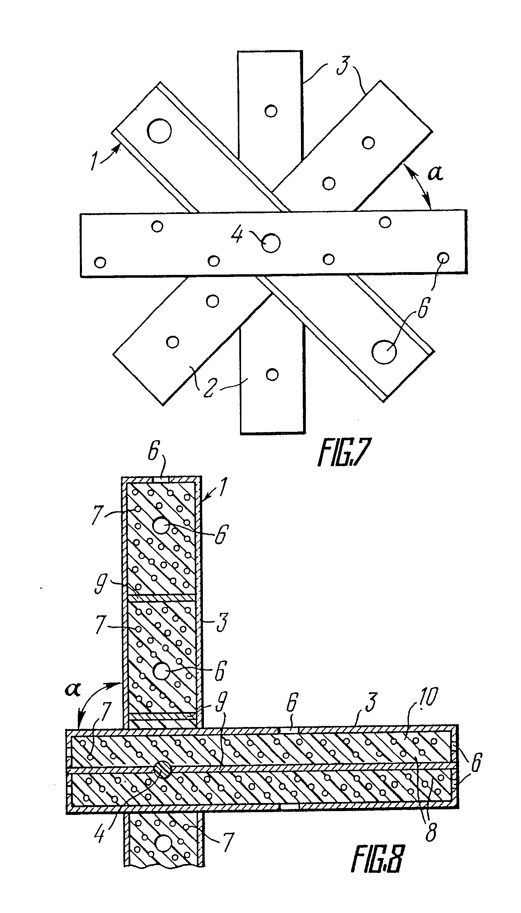

In Fig. 2 ist der Querschnitt nach der Linie II-II aus der Fig. I der Einrichtung zur kontrollierbaren dosierten Einführung von Wirkstoffen in das Funktionierungsmedium dargestellt. Die Teile 3 des Gehäuses I sind in bezug auf das Funktionierungsmedium hermetisch abgeschlossen. Im Arbeitszustand nimmt die Einrichtung zur kontrollierbaren dosierten Einführung von Wirkstoffen eine Form an, die das Festhalten dieser Konstruktion im Tiermagen bewirkt, wobei die Teile 3 (Fig. 3) des Gehäuses I relativ zueinander unter einem Winkel $\propto$ gleich 90° orientiert sind. In Fig. 4 ist die Einrichtung zur kontrollierbaren dosierten Einführung von Wirkstoffen im Querschnitt nach der Linie 1V-IV aus der Fig. 3 dargestellt. In jedem Teil 3 des Gehäuses I sind verschiedene Wirkstoffe 7 untergebracht. So ist in einem der Teile 3 in einer polymeren Matrize 8 aus Polyvinylchlorid-Plastisol als Wirkstoff 7 Tividin, in dem anderen Teil 3 desselben aber in einer aus einem Mischpolymerisat von Vinylazetat-Äthylen bestehenden Matrize 8 als Wirkstoff 7 ein Antiseptikum, nämlich Zinksulfat, enthalten.

Das zylinderförmige Gehäuse I (Fig. 5) der Einrichtung kann trennbar mit zwei Trennungsebenen ausgeführt sein, und eine Trennwand 2 trennt dasselbe in drei Teile 3. Dabei erfolgt eine Drehung der Teile 3 um einen Winkel $\propto$ gleich 60°. Die Teile 3 des Gehäuses I sind mit einem Einsatzstück 9 versehen, das den Innenraum eines jeden Teiles 3 des Gehäuses I in Abteilungen I0 unterteilt. Dies gestattet es, verschiedenartige Wirkstoffe 7 aus jeder Abteilung I0 des Teiles 3 des Gehäuses I einzuführen. Beispielsweise befindet sich in einer Abteilung I0 des Teiles 3 als Wirkstoff 7 Glukose, während in einer anderen Kaliumjodid enthalten ist. Die Abteilungen I0 sind voneinander isoliert. Das Einsatzstück 9 ist an den Innenwänden des Teiles 3 befestigt und liegt in einer zur Längsachse des Gehäuses I senkrechten Ebene. Das zylindrische Gehäuse I (Fig. 6) kann auch trennbar mit drei Trennungsebenen ausgeführt sein, wobei die Trennwand 2 das Gehäuse I in vier Teile 3 trennt. In jedem Teil 3 des Gehäuses I kann ein und derselbe Wirkstoff 7 enthalten sein, es können aber auch verschiedene Wirkstoffe 7, wie es im vorliegenden Fall geschehen ist, in einzelne Gehäuseteile eingebracht sein. Beispielsweise ist in einem Teil 3 Tividin, in einem anderen - Zinksulfat, in einem dritten - Glukose, in einem vierten Vitatetrin enthalten. Die Fixatoren 5 halten das zylindrische Gehäuse I im zusammengebauten Zustand bis zum Augenblick des Einbringens des Gehäuses I in das Funktionierungsmedium fest. Die Verbindung der Teile des Gehäuses I kann durch Auftragen einer konzentrierten Lösung von Polyvinylalkohol auf die Trennwände 2 vorgenommen werden, wobei der Polyvinylalkohol das Gehäuse I im zusammengebauten Zustand bis zum Augenblick des Einbringens desselben in das Funktionierungsmedium festhält. Im Augenblick des Einbringens des Gehäuses I in das Funktionierungsmedium werden die Teile 3 (Fig. 7) des Gehäuses I relativ zueinander in der zur Längsachse des Gehäuses I parallelen Ebene um einen Winkel ∝ = 45° gedreht. In den Wänden der Teile 3 des Gehäuses I sind Bohrungen 6 ausgeführt, die die Mittel zum Verbinden des Gehäuses I mit dem Funktionierungsmedium darstellen. Die Abmessungen der zur Einführung in den Tiermagen bestimmten Einrichtung sind durch die Speiseröhrenmaße begrenzt. Die optimale Anzahl von Teilen 3 des Gehäuses I ist vier. Wird die Einrichtung in den Magen von Schafen und anderen kleinen Wiederkäuern eingeführt, so darf die Anzahl der Teile 3 zwei nicht übersteigen. Beim Einführen der Wirkstoffe 7 in Wasserteiche von Fischzuchtbetrieben soll die Einrichtung grössere Abmessungen besitzen, wobei das Gehäuse I in Teile 3 getrennt ist, deren Anzahl grösser als vier ist.

Die Einrichtung zur kontrollierbaren dosierten Einführung von Wirkstoffen kann in einem Teil 3 (Fig. 8) des Gehäuses I ein Einsatzstück 9 enthalten, das in einer zur Längsachse des Gehäuses I parallelen Ebene liegt. Das Einsatzstück 9 unterteilt diesen Teil 3 in zwei Abteilungen I0. Im zweiten Teil 3 des Gehäuses I sind zwei Einsatzstücke 9 vorhanden, die in einer zur Längsachse des Gehäuses I senkrechten Ebene liegen. Diese Einsatzstücke 9 unterteilen den Teil 3 des Gehäuses I in drei voneinander isolierte Abteilungen I0. Dabei erfolgt die Drehung der Teile 3 des Gehäuses I um einen Winkel ∝ gleich 90°. In den Abteilungen I0 der Teile 3 befinden sich verschiedene Wirkstoffe 7. Beispielsweise befindet sich in einer Abteilung I0 des durch das Einsatzstück 9 in zwei Abteilungen I0 unterteilten Teiles 3 Tividin, in der anderen Abteilung I0 aber Glukose. Im zweiten Teil 3 ist in einer Abteilung I0 Vitatetrin, in der anderen Abteilung I0 Natriumchlorid, in einer nächsten Abteilung I0 Zinksulfat enthalten. Die Drehung der Teile 3 des Gehäuses I wird zweckdienlicherweise um einen Winkel ausgeführt, der innerhalb einer Spanne von I0 bis 90° liegt. Bei einer Winkelgrösse von weniger als I0° wird das Festhalten der Einrichtung, beispielsweise im Tiermagen während der gesamten Dauer der Einführung der Wirkstoffe 7 in das Funktionierungsmedium nicht gewährleistet. Bei einer Winkelgrösse von über 90° nehmen die von der Torsionsfeder 4 erzeugten Kräfte stark zu, und somit wird auch die Dicke der Fixatoren 5 grösser. Darüber hinaus wird das Festhalten der Einrichtung beispielsweise im Tiermagen während der gesamten Dauer der Einführung der Wirkstoffe 7 in das Funktionierungsmedium nicht gewährleistet.

Der Wirkstoff 7 kann im Innern des Gehäuses I auch ohne eine polymere Matrize 8, beispielsweise in Form von Pulver, gepresster oder eingeschmolzener Masse untergebracht sein.

Die Einrichtung zur kontrollierbaren dosierten Einführung von Wirkstoffen in das Funktionierungsmedium arbeitet wie folgt.

Vor dem Einbringen der Einrichtung in das Funktionierungsmedium, im vorliegenden Beispiel in den Magen eines Wiederkäuers, werden die Teile 3 (Fig. I) des Gehäuses I aneinandergepasst und in dieser Stellung mit Hilfe von Fixatoren 5 fixiert. Danach wird die Einrichtung in das Funktionierungsmedium eingebracht, wo unter der Einwirkung des flüssigen Mediums die Zerstörung der Fixatoren 5 erfolgt und unter der Einwirkung der Torsionsfeder 4 die Teile 3 (Fig. 3) des Gehäuses I relativ zueinander bis zu einem Winkel von 90° gedreht werden. Dies bewirkt das Festhalten der Einrichtung im Tiermagen während der gesamten Funktionsdauer derselben. Die Ausführung des Gehäuses I in trennbarer Form mit relativ zueinander in zur Längsachse des Gehäuses I parallelen Ebenen gedreht an Teilen 3 sowie das Fehlen von zusätzlichen Fixatoren ermöglicht es, die Wahrscheinlichkeit einer Verletzung von Magenwänden zu verringern.

Bei einer Verschiebung der Einrichtung im Funktionierungsmedium strömt die Flüssigkeit des Funktionierungsmediums über die Bohrungen 6 zum in der polymeren Matrize 8 enthaltenen Wirkstoff 7, löst den Wirkstoff 7 auf, der nun in Form einer Lösung über die Bohrungen 6 in das Funktionierungsmedium tritt. Dabei werden aus jedem einzelnen Teil 3 (Fig. 4) des Gehäuses I unterschiedliche Wirkstoffe 7 dosiert: aus dem einen Teil 3 - Tividin, aus dem anderen - Zinksulfat. Dies ist dank einer rationellen Nutzung des Innenraums des Gehäuses I möglich, wenn ein jeder Teil 3 des Gehäuses I ein isoliertes Volumen mit einem Wirkstoff 7 darstellt. Die Einrichtung bewirkt die Dosierung eines jedem von den Wirkstoffen 7 nach einem vorgegebenen Verlauf innerhalb einer Zeitspanne von höchstens 200 Tagen.

Ähnlicherweise arbeitet auch die Einrichtung, die die Dosierung von gleichzeitig mehr als zwei Wirkstoffen 7 (Fig. 5) ermöglicht. Das in jedem der drei Teile 3 des Gehäuses I vorhandene Einsatzstück 9 gestattet es, in jedem Teil 3 zwei voneinander isolierte Abteilungen I0 zu schaffen, aus denen verschiedene Wirkstoffe 7 nach dem jeweils vorgeschriebenen Dosierungsgesetz dosiert werden. Dadurch gestattet es die Einrichtung dank einer rationellen Nutzung des Innenraums des Gehäuses I und der Schaffung von isolierten Abteilungen I0 mit in denselben enthaltenem Wirkstoff 7, gleichzeitig bis zu sechs miteinander nicht vermischbare Wirkstoffe 7 einzuführen.

Beim Unterteilen des Gehäuses I durch die Trennwand 2 (Fig. 6) in vier Teile 3 werden aus jedem Teil 3 in das Funktionierungsmedium vier verschiedene Wirkstoffe 7 eingeführt: Tividin, Zinksulfat, Glukose, Vitatetrin.

Ist ein Teil 3 (Fig. 8) durch die Trennwand 9 in zwei Abteilungen I0, der andere Teil 3 aber durch die Trennwand 9 in drei Abteilungen I0 unterteilt, so ermöglicht die vorliegende Einrichtung die gleichzeitige Einführung von fünf verschiedenen Wirkstoffen 7: Vitatetrin, Natriumchlorid, Zinksulfat, Tividin, Glukose.

Somit gestattet es die vorliegende Erfindung dank der Ausführung des zylindrischen Gehäuses I mit trennbaren Teilen 3, die in bezug auf das Funktionierungsmedium hermetisch abgeschlossen und relativ zueinander in der zur Längsachse des Gehäuses I parallelen Ebene gedreht sind, sowie dank dem Ausstatten der Teile 3 mit Einsatzstükken 9, die den Innenraum der Teile 3 in voneinander isolierte Abteilungen I0 unterteilen, eine prolongierte dosierte Einführung von gleichzeitig mehreren Wirkstoffen 7 vorzunehmen sowie die Wahrscheinlichkeit einer Beschädigung der Tierpansenwände zu verringern.

Industrielle Verwertbarkeit

Die Erfindung kann als eine Antihelminthenrichtung zur Ausheilung und Prophylaxe bei während der sommerlichen Weidezeit zur Mast getriebenen Tieren angewendet werden. Darüber hinaus kann die Erfindung in der Medizin, in der chemischen und Fotoindustrie angewendet werden.

**Ansprüche**

1. Einrichtung zur kontrollierbaren dosierten Einführung von Wirkstoffen in ein Funktionierungsmedium, die ein hohles zylindrisches Gehäuse mit einem Mittel zum Verbinden desselben mit dem Funktionierungsmedium enthält, dadurch **gekennzeichnet**, dass ein zylindrisches Gehäuse (I) trennbar mit mindestens einer Trennungsebene ausgeführt ist, die entlang einer Mantellinie des zylindrischen Gehäuses (I)-verläuft und in der sich eine Trennwand (2) befindet, die das zylindrische Gehäuse (I) in Teile (3) trennt, die in bezug auf das Funktionierungsmedium hermetisch abgeschlossen sind, wobei jeder der Teile (3) des zylindrischen Gehäuses (I) mindestens ein Mittel zum Verbinden desselben mit dem Funktionierungsmedium besitzt und die Teile (3) des zylindrischen Gehäuses (I) durch eine Torsionsfeder (4) miteinander verbunden sind, deren Achse zur Längsachse des zylindrischen Gehäuses (I) senkrecht und die mit der Möglichkeit einer Drehung der Teile (3) des zylindrischen Gehäuses (I) relativ zueinander in der zur Längsachse des zylindrischen Gehäuses (I) parallelen Ebene im Augenblick des Einbringens des zylindrischen Gehäuses (I) in das Funktionierungsmedium angebracht ist.

2. Einrichtung zur kontrollierbaren dosierten Einführung von Wirkstoffen in ein Funktionierungsmedium nach Anspruch 1, dadurch **gekennzeichnet**, dass sie mit zumindest einem Fixator (5) aus einem löslichen Material ausgestattet ist, der an der Aussenfläche des zylindrischen Gehäuses (I) angebracht ist und die Teile des zylindrischen Gehäuses (I) im zusammengebauten Zustand bis zum Augenblick des Einbringens des zylindrischen Gehäuses (I) in das Funktionierungsmedium festhält.

3. Einrichtung zur kontrollierbaren dosierten Einführung von Wirkstoffen in ein Funktionierungsmedium nach Anspruch 2, dadurch **gekennzeichnet**, dass die Drehung der Teile (3) des zylindrischen Gehäuses (I) um einen Winkel ($\propto$) erfolgt, der innerhalb einer Spanne von I0 bis 90° liegt.

4. Einrichtung zur kontrollierbaren dosierten Einführung von Wirkstoffen in ein Funktionierungsmedium nach Anspruch I oder nach Anspruch 2 oder 3, dadurch **gekennzeichnet**, dass jeder Teil (3) des zylindrischen Gehäuses (I) mit mindestens einem Einsatzstück (9) ausgestattet ist, das sich im Innern des Teiles (3) oder des zylindrischeh Gehäuses (I) befindet und an den Innenwänden desselben befestigt ist, wobei das Einsatzstück (9) den Innenraum des Teiles (3) des zylindrischen Gehäuses (I) in voneinander isolierte Abteilungen (I0) unterteilt.

5. Einrichtung zur kontrollierbaren dosierten Einführung von Wirkstoffen in ein Funktionierungsmedium nach Anspruch 4, dadurch **gekennzeichnet**, dass das Einsatzstück (9) in der zur Längsachse des zylindrischen Gehäuses (I) senkrechten Ebene liegt.

6. Einrichtung zur kontrollierbaren dosierten Einführung von Wirkstoffen in ein Funktionierungsmedium nach Anspruch 4, dadurch **gekennzeichnet**, dass das Einsatzstück (9) in einer zur Längsachse des zylindrischen Gehäuses (I) parallelen Ebene liegt.

7. Einrichtung zur kontrollierbaren dosierten Einführung von Wirkstoffen in ein Funktionierungsmedium nach Anspruch I oder Anspruch 2 oder 3, dadurch **gekennzeichnet**, dass das zylindrische Gehäuse (I) trennbar mit einer Trennungsebene ausgeführt ist, wobei die Trennwand (2) das zylindrische Gehäuse (I) in zwei Teile (3) trennt und eine Drehung derselben um einen Winkel ( $\propto$ ) von 90° ausführbar ist.

8. Einrichtung zur kontrollierbaren dosierten Einführung von Wirkstoffen in ein Funktionierungsmedium nach Anspruch I oder 2 oder 3, dadurch **gekennzeichnet**, dass das zylindrischen Gehäuse (I) trennbar mit zwei Trennungsebenen ausgeführt ist, wobei die Trennwand (2) das zylindrische Gehäuse (I) in drei Teile (3) trennt und eine Drehung derselben um einen Winkel ( $\propto$ ) von 60° ausführbar ist.

9. Einrichtung zur kontrollierbaren dosierten Einführung von Wirkstoffen in ein Funktionierungsmedium nach Anspruch I oder Anspruch 2 oder 3, dadurch **gekennzeichnet**, dass das zylindrische Gehäuse (I) trennbar mit drei Trennungsebenen ausgeführt ist, wobei die Trennwand (2) das zylindrische Gehäuse (I) in vier Teile (3) trennt und eine Drehung derselben um einen Winkel ( $\propto$ ) von 45° ausführbar ist.

10. Einrichtung zur kontrollierbaren dosierten Einführung von Wirkstoffen in ein Funktionierungsmedium nach Anspruch 4, dadurch **gekennzeichnet**, dass das zylindrische Gehäuse (I) trennbar mit einer Trennungsebene ausgeführt ist, wobei die Trennwand (2) das zylindrische Gehäuse (I) in zwei Teile (3) trennt und eine Drehung derselben um einen Winkel ( $\propto$ ) von 90° ausführbar ist.

11. Einrichtung zur kontrollierbaren dosierten Einführung von Wirkstoffen in ein Funktionierungsmedium nach Anspruch 4, dadurch **gekennzeichnet** dass das zylindrische Gehäuse (I) trennbar mit zwei Trennungsebenen ausgeführt ist, wobei die Trennwand (2) das zylindrische Gehäuse (I) in drei Teile (3) trennt und eine Drehung der selben um einen Winkel ( $\propto$ ) von 60° ausführbar ist.

12. Einrichtung zur kontrollierbaren dosierten Einführung von Wirkstoffen in ein Funktionierungsmedium nach Anspruch 4, dadurch **gekennzeichnet**, dass das zylindrische Gehäuse (I) trennbar mit drei Trennungsebenen ausgeführt ist, wobei die Trennwand (2) das zylindrische Gehäuse (I) in vier Teile (3) trennt und eine Drehung der selben um einen Winkel ( $\propto$ ) von

45° ausführbar ist.

FIG.1

FIG.2

FIG.3

FIG.4

FIG.5

FIG.6

11

FIG.7

FIG.8

# INTERNATIONAL SEARCH REPORT

International Application No PCT/SU  89/00109

## I. CLASSIFICATION OF SUBJECT MATTER (if several classification symbols apply, indicate all)

According to International Patent Classification (IPC) or to both National Classification and IPC

Int. Cl.$^5$   A 61 K   9/52

## II. FIELDS SEARCHED

### Minimum Documentation Searched [7]

| Classification System | Classification Symbols |
|---|---|
| Int. Cl.$^4$ | A 61 K 9/22 – 9/52, A 61 M 31/00 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | US, A, 4416659 (Eli Lilly and Company), 22 November 1983 (22.11.83), the abstract, the claims<br>      &    EP 79724<br>            GB 2109232<br>            JP 58088310 | 1-12 |
| A | US, A, 4612008 (Alza Corporation), 16 September 1986 (16.09.86), the abstract, figures 5-7<br>      &    GB 2140687<br>            DE 3417113<br>            FR 2545721<br>            JP 60041609 | 1-4 |

./.

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 28 NOvember 1989 (28.11.89) | 8 January 1990 (08.01.90) |

| International Searching Authority | Signature of Authorized Officer |
|---|---|
| ISA/SU | |

Form PCT/ISA/210 (second sheet) (January 1985)